(19) Europäisches Patentamt / European Patent Office / Office européen des brevets

(11) **EP 2 847 138 B1**

(12) **EUROPÄISCHE PATENTSCHRIFT**

(45) Veröffentlichungstag und Bekanntmachung des Hinweises auf die Patenterteilung:
**04.07.2018 Patentblatt 2018/27**

(21) Anmeldenummer: **13724202.0**

(22) Anmeldetag: **10.05.2013**

(51) Int Cl.:
*C03C 3/083* (2006.01)   *C03C 3/085* (2006.01)
*C03C 3/095* (2006.01)   *C03C 3/097* (2006.01)
*C03C 10/00* (2006.01)   *C03C 4/00* (2006.01)
*A61C 13/08* (2006.01)   *A61C 13/083* (2006.01)
*A61K 6/02* (2006.01)   *C03B 32/02* (2006.01)
*C04B 35/16* (2006.01)   *A61C 13/00* (2006.01)

(86) Internationale Anmeldenummer:
**PCT/EP2013/059700**

(87) Internationale Veröffentlichungsnummer:
**WO 2013/167723 (14.11.2013 Gazette 2013/46)**

(54) **VORGESINTERTER ROHLING FÜR DENTALE ZWECKE**

PRE-SINTERED BLANK FOR DENTAL PURPOSES

ÉBAUCHE PRÉALABLEMENT FRITTÉE À DES FINS DENTAIRES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **11.05.2012 EP 12167760**

(43) Veröffentlichungstag der Anmeldung:
**18.03.2015 Patentblatt 2015/12**

(73) Patentinhaber: **Ivoclar Vivadent AG**
**9494 Schaan (LI)**

(72) Erfinder:
• **BÜRKE, Harald**
**A-6820 Frastanz (AT)**

• **RITZBERGER, Christian**
**CH-9472 Grabs (CH)**
• **SCHWEIGER, Marcel**
**CH-7000 Chur (CH)**
• **RHEINBERGER, Volker**
**CH-9490 Vaduz (LI)**

(74) Vertreter: **Uexküll & Stolberg**
**Partnerschaft von**
**Patent- und Rechtsanwälten mbB**
**Beselerstraße 4**
**22607 Hamburg (DE)**

(56) Entgegenhaltungen:
**EP-A1- 1 505 041   EP-A1- 1 688 398**
**DE-A1- 2 451 121   US-B1- 6 455 451**

EP 2 847 138 B1

**Beschreibung**

Die Erfindung betrifft einen vorgesinterten Rohling für dentale Zwecke auf Basis von Lithiumdisilikat-Glaskeramik, der sich insbesondere für die Herstellung von Dentalrestaurationen eignet.

Über die Verwendung von vorgesinterten Rohlingen in der Dentaltechnik ist bereits im Stand der Technik berichtet worden.

Die WO 2010/010087 beschreibt poröse silikatkeramische Formkörper, die zu Verblendschalen für Dentaltechnik verarbeitet werden. Die Formkörper sollen dabei eine bestimmte Dichte haben, um Beschädigungen bei der Bearbeitung mit Fräs- oder Schleifsystemen z.B. durch Ausbrüche von Material zu verhindern und für das ausgewählte System geeignet zu sein.

Die US 5,106,303 beschreibt die Herstellung von Zahnkronen und Inlays durch Kopierschleifen von kompaktierten keramischen Körpern, die gegebenenfalls vorgesintert sein können. Zur Erzielung der gewünschten Geometrie werden die Körper auf eine vergrößerte Form geschliffen, um die Schwindung zu berücksichtigen, die bei der anschließenden Sinterung zu der angestrebten hohen Dichte auftritt. Als keramisches Material wird insbesondere Aluminiumoxid benutzt, welches gegebenenfalls verstärkende Zusätze aufweisen kann.

Die US 5,775,912 beschreibt vorgesinterte Pellets aus Dentalporzellan, aus denen mittels CAD/CAM-Systemen eine Zahnstruktur geschliffen wird. Diese Zahnstruktur wird in Einbettmaterial eingebettet, gesintert und von dem Einbettmaterial befreit, um die gewünschte Dentalrestauration zu ergeben. Bei den eingesetzten Dentalporzellanen handelt es sich um Glaskeramiken auf Basis von Leucit.

Die US 6,354,836 offenbart Verfahren zur Herstellung von dentalen Restaurationen unter Verwendung von CAD/CAM-Methoden. Es werden dafür ungesinterte oder vorgesinterte Blöcke aus keramischem Material und insbesondere Aluminiumoxid und Zirkoniumoxid eingesetzt, die nach Schleifen auf eine vergrößerte Form und anschließendes Dichtsintern zu hochfesten Dentalrestaurationen führen. Dabei wird es allerdings als wesentlich erachtet, dass die Temperaturunterschiede im eingesetzten Sinterofen kleiner als 10°C sind, um für geringe Schwankungen bei den schließlich erzielten Abmessungen der Restaurationen zu sorgen.

Bei den bekannten vorgesinterten Rohlingen ist die beim Dichtsintern auftretende Schwindung und damit der jeweils anzuwendende Vergrösserungsfaktor in hohem Maße von der angewendeten Vorsintertemperatur abhängig. Bereits kleine Abweichungen, wie sie infolge einer inhomogenen Temperaturverteilung im Sinterofen auftreten können, führen zu unterschiedlichen Schwindungen beim Dichtsintern. Diese Schwankungen erlauben jedoch nicht die angestrebten geringen Toleranzen bei den Abmessungen der erzeugten Dentalrestauration.

Der Erfindung liegt daher die Aufgabe zugrunde, vorgesinterte Rohlinge zur Verfügung zu stellen, die diese Nachteile vermeiden und daher gegenüber Schwankungen bei der zu ihrer Herstellung angewendeten Sintertemperatur weniger empfindlich sind. Gleichfalls sollen diese Rohlinge einfach mittels üblicher Schleif- und Fräsverfahren zu Dentalrestaurationen mit der gewünschten Geometrie geformt werden können, ohne dass bei diesen Verfahren die Zufuhr von Flüssigkeit erforderlich wäre. Weiter sollen diese Rohlinge durch Dichtsintern zu hochfesten und optisch sehr ansprechenden Dentalrestaurationen verarbeitbar sein.

Diese Aufgabe wird durch den vorgesinterten Rohling nach den Ansprüchen 1 bis 9 gelöst. Gegenstand der Erfindung ist ebenfalls das Verfahren zur Herstellung des Rohlings nach Anspruch 10 und 11, das Verfahren zur Herstellung von Dentalrestaurationen nach den Ansprüchen 12 bis 15 sowie die Verwendung des Rohlings nach Anspruch 16.

Der erfindungsgemäße vorgesinterte Rohling für dentale Zwecke zeichnet sich dadurch aus, dass er auf Basis von Lithiumdisilikat-Glaskeramik ist und eine relative Dichte von 60 bis 90%, insbesondere 62 bis 88% und bevorzugt 65 bis 87 %, bezogen auf die Reindichte der Glaskeramik, aufweist.

Die relative Dichte ist dabei das Verhältnis von der Dichte des vorgesinterten Rohlings zur Reindichte der Glaskeramik.

Die Dichte des vorgesinterten Rohlings wird bestimmt, indem dieser gewogen und sein Volumen geometrisch ermittelt wird. Die Dichte wird dann nach der bekannten Formel

```
Dichte = Masse / Volumen
```

berechnet.

Die Bestimmung der Reindichte der Glaskeramik erfolgt, indem der vorgesinterte Rohling auf ein Pulver mit einer mittleren Teilchengrösse von 10 bis 30 $\mu$m, insbesondere von 20 $\mu$m, bezogen auf die Anzahl der Teilchen, gemahlen und die Dichte des Pulvers mittels Pyknometer ermittelt wird. Die Bestimmung der Teilchengrösse wurde mit dem CILAS® Particle Size Analyzer 1064 der Firma Quantachrome GmbH & Co. KG mittels Laserbeugung nach ISO 13320 (2009) durchgeführt.

Es hat sich überraschenderweise herausgestellt, dass der erfindungsgemäße Rohling nicht nur in einfacher Weise maschinell trocken bearbeitet werden kann, sondern auch bei sich signifikant unterscheidenden Vorsintertemperaturen hergestellt werden kann, ohne dass dies zu einer wesentlichen Veränderung der Schwindung führen würde, die bei einem anschließenden Dichtsintern auftritt. Damit kann der die auftretende Schwindung berücksichtigende Vergrößerungsfaktor sehr genau bestimmt werden. Diese vorteilhaften Eigenschaften sind offenbar auf das besondere Verhalten von Lithiumdisilikat-Glaskeramik zurückzuführen, welche auf die oben angegebenen relativen Dichten vorgesintert wurde.

Es ist weiter bevorzugt, dass der Rohling im Wesentlichen aus der Lithiumdisilikat-Glaskeramik besteht. Besonders bevorzugt besteht der Rohling aus der Lithiumdisilikat-Glaskeramik.

Die Glaskeramik weist in einer bevorzugten Ausführungsform Lithiumdisilikat als Hauptkristallphase auf. Dabei bezeichnet der Begriff "Hauptkristallphase" die Kristallphase, die gegenüber anderen Kristallphasen den höchsten Volumenanteil hat. Insbesondere enthält die Glaskeramik mehr als 10 Vol.-%, bevorzugt mehr als 20 Vol.-% und besonders bevorzugt mehr als 30 Vol.-% an Lithiumdisilikat-Kristallen, bezogen auf die gesamte Glaskeramik.

Die Lithiumdisilikat-Glaskeramik enthält $SiO_2$ und $Li_2O$ vorzugsweise in einem Molverhältnis im Bereich von 1,75 bis 3,0, insbesondere 1,8 bis 2,6.

In einer weiteren bevorzugten Ausführungsform enthält die Lithiumdisilikat-Glaskeramik mindestens eine der folgenden Komponenten:

| Komponente | Gew.-% |
|---|---|
| $SiO_2$ | 50,0 bis 80,0 |
| $Li_2O$ | 6,0 bis 20,0 |
| $Me(I)_2O$ | 0 bis 10,0, insbesondere 0,1 bis 10,0 |
| $Me(II)O$ | 0 bis 12,0, insbesondere 0,1 bis 12,0 |
| $Me(III)_2O_3$ | 0 bis 8,0, insbesondere 0,1 bis 8,0 |
| $Me(IV)O_2$ | 0 bis 8,0, insbesondere 0,1 bis 8,0 |
| $Me(V)_2O_5$ | 0 bis 8,0, insbesondere 0,1 bis 8,0 |
| $Me(VI)O_3$ | 0 bis 8,0, insbesondere 0,1 bis 8,0 |
| Keimbildner | 0 bis 8,0, insbesondere 0,1 bis 8,0 |

wobei

$Me(I)_2O$ ausgewählt ist aus $Na_2O$, $K_2O$, $Rb_2O$, $Cs_2O$ oder Mischungen davon,

$Me(II)O$ ausgewählt ist aus CaO, BaO, MgO, SrO, ZnO und Mischungen davon,

$Me(III)_2O_3$ ausgewählt ist aus $Al_2O_3$, $La_2O_3$, $Bi_2O_3$, $Y_2O_3$, $Yb_2O_3$ und Mischungen davon,

$Me(IV)O_2$ ausgewählt ist aus $ZrO_2$, $TiO_2$, $SnO_2$, $GeO_2$ und Mischungen davon,

$Me(V)_2O_5$ ausgewählt ist aus $Ta_2O_5$, $Nb_2O_5$, $V_2O_5$ und Mischungen davon,

$Me(VI)O_3$ ausgewählt ist aus $WO_3$, $MoO_3$ und Mischungen davon, und

Keimbildner ausgewählt ist aus $P_2O_5$, Metallen und Mischungen davon.

Als Oxide einwertiger Elemente $Me(I)_2O$ sind $Na_2O$ und $K_2O$ bevorzugt.
Als Oxide zweiwertiger Elemente $Me(II)O$ sind CaO, MgO, SrO und ZnO bevorzugt.
Als Oxide dreiwertiger Elemente $Me(III)_2O_3$ sind $Al_2O_3$, $La_2O_3$ und $Y_2O_3$ bevorzugt.
Als Oxide vierwertiger Elemente $Me(IV)O_2$ sind $ZrO_2$, $TiO_2$ und $GeO_2$ bevorzugt.
Als Oxide fünfwertiger Elemente $Me(V)_2O_5$ sind $Ta_2O_5$ und $Nb_2O_5$ bevorzugt.
Als Oxide sechswertiger Elemente $Me(VI)O_3$ sind $WO_3$ und $MoO_3$ bevorzugt.
Als Keimbildner ist $P_2O_5$ bevorzugt.
Die Lithiumdisilikat-Glaskeramik enthält bevorzugt Färbemittel und/oder Fluoreszenzmitteln.
Beispiele für Färbemittel und Fluoreszenzmittel sind anorganische Pigmente und/oder Oxide von d- und f-Elementen, wie z.B. die Oxide von Ti, V, Sc, Mn, Fe, Co, Ta, W, Ce, Pr, Nd, Tb, Er, Dy, Gd, Eu und Yb. Als Färbemittel können auch Metallkolloide, z.B. von Ag, Au und Pd, verwendet werden, die zusätzlich auch als Keimbildner fungieren können. Diese Metallkolloide können z.B. durch Reduktion von entsprechenden Oxiden, Chloriden oder Nitraten während der Schmelz- und Kristallisationsprozesse gebildet werden. Als anorganische Pigmente werden beispielsweise dotierte Spinelle, Zirkonsilikat, Stannate, dotierter Korund und/oder dotiertes $ZrO_2$ eingesetzt.

Der erfindungsgemäße Rohling weist vorzugsweise mindestens zwei Bereiche, insbesondere Schichten, auf, die sich durch ihre Färbung oder Transluzenz unterscheiden. Bevorzugt hat der Rohling mindestens 3 und bis zu 10, besonders bevorzugt mindestens 3 und bis zu 8, und ganz besonders bevorzugt mindestens 4 und bis zu 6 sich in Färbung oder Transluzenz unterscheidende Bereiche, insbesondere Schichten. Gerade durch das Vorliegen von mehreren unterschiedlich gefärbten Bereichen, insbesondere Schichten, gelingt es sehr gut, natürliches Zahnmaterial zu imitieren. Es ist ebenfalls möglich, dass wenigstens einer der Bereiche oder der Schichten einen Farbgradienten aufweist, um einen stufenlosen Farbübergang zu gewährleisten.

In einer weiteren bevorzugten Ausführungsform weist der erfindungsgemäße Rohling einen Halter für die Befestigung in einer Bearbeitungsvorrichtung auf. In einer anderen bevorzugten Ausführungsform hat der erfindungsgemäße Rohling eine Schnittstelle zur Verbindung mit einem Dentalimplantat.

Der Halter gestattet die Befestigung des Rohlings in einer Bearbeitungsvorrichtung, wie insbesondere einer Fräs- oder Schleifvorrichtung. Der Halter hat üblicherweise die Form eines Stifts und besteht vorzugsweise aus Metall oder Kunststoff.

Die Schnittstelle sorgt für eine Verbindung zwischen einem Implantat und der darauf aufgesetzten Dentalrestauration, wie insbesondere Abutmentkrone, die durch maschinelle Bearbeitung und Dichtsinterung des Rohlings erhalten worden ist. Diese Verbindung ist bevorzugt rotationsfest. Die Schnittstelle liegt insbesondere in Form einer Ausnehmung, wie einer Bohrung, vor. Die konkrete Geometrie der Schnittstelle wird dabei üblicherweise in Abhängigkeit von dem jeweils verwendeten Implantatsystem gewählt.

Die Erfindung betrifft ebenfalls ein Verfahren zur Herstellung des erfindungsgemäßen Rohlings, bei dem

(a) Lithiumsilikat-Glas in Pulver- oder Granulatform zu einem Glasrohling verpresst wird,
(b) der Glasrohling wärmebehandelt wird, um einen vorgesinterten Rohling auf Basis von Lithiumdisilikat-Glaskeramik herzustellen, wobei die Temperatur der Wärmebehandlung

(i) mindestens 500°C, insbesondere mindestens 540°C und bevorzugt mindestens 580°C beträgt, und

(ii) in einem Bereich liegt, der sich über mindestens 30K, insbesondere mindestens 50K und bevorzugt mindestens 70K erstreckt und in dem die relative Dichte um weniger als 2.5 %, insbesondere weniger als 2.0 % und bevorzugt weniger als 1.5 % variiert.

In Stufe (a) wird Lithiumsilikat-Glas in Pulver- oder Granulatform zu einem Glasrohling verpresst.

Das dabei eingesetzte Lithiumsilikatglas wird üblicherweise hergestellt, indem eine Mischung von geeigneten Ausgangsmaterialien, wie z.B. Carbonaten, Oxiden, Phosphaten und Fluoriden, bei Temperaturen von insbesondere 1300 bis 1600°C für 2 bis 10 h erschmolzen wird. Zur Erzielung einer besonders hohen Homogenität wird die erhaltene Glasschmelze in Wasser gegossen, um ein Glasgranulat zu bilden, und das erhaltene Granulat wird dann erneut aufgeschmolzen.

Das Granulat wird dann auf die gewünschte Teilchengröße zerkleinert und insbesondere zu Pulver mit einer mittleren Teilchengröße von < 100 $\mu$m, bezogen auf die Anzahl der Teilchen, gemahlen.

Das Granulat oder Pulver wird dann, gegebenenfalls unter Zusatz von Presshilfsmitteln oder Bindemitteln, üblicherweise in eine Pressform gegeben und zu einem Glasrohling verpresst. Der dabei angewendete Druck liegt insbesondere im Bereich von 20 bis 200 MPa. Für das Verpressen werden bevorzugt Uniaxial-Pressen eingesetzt. Das Verpressen kann insbesondere auch als isostatisches Pressen, bevorzugt als kalt-isostatisches Pressen durchgeführt werden.

Durch Verwendung von Glaspulvern oder -granulaten mit unterschiedlicher Färbung oder Transluzenz können Glasrohlinge erzeugt werden, die unterschiedlich gefärbte oder unterschiedlich transluzente Bereiche und insbesondere Schichten aufweisen. Beispielsweise können verschieden gefärbte Pulver oder Granulate in einer Pressform übereinander angeordnet werden, so dass ein mehrfarbiger Glasrohling erzeugt wird. Diese Mehrfarbigkeit gestattet es in hohem Maße, den schließlich hergestellten Dentalrestaurationen das Aussehen von natürlichem Zahnmaterial zu verleihen.

In Stufe (b) wird der erhaltene ein- oder mehrfarbige Glasrohling einer Wärmebehandlung unterzogen, um die gesteuerte Kristallisation von Lithiumdisilikat und damit die Bildung einer Lithiumdisilikat-Glaskeramik sowie die Vorsinterung herbeizuführen. Die Wärmebehandlung findet insbesondere bei einer Temperatur von 500°C bis 900°C, bevorzugt 540 bis 900°C und besonders bevorzugt 580 bis 900°C statt. Die Wärmebehandlung wird insbesondere für eine Dauer von 2 bis 120 min, bevorzugt 5 bis 60 min und besonders bevorzugt 10 bis 30 min, durchgeführt.

Der Temperaturbereich (b)(ii) beschreibt einen Bereich, in dem trotz Änderung der Temperatur sich die relative Dichte kaum ändert. Dieser Bereich wird daher im Folgenden auch als "Plateau" bezeichnet. Die in diesem Bereich mögliche Variation der relativen Dichte errechnet sich in % aus dem Maximal- und Minimalwert der relativen Dichte in

dem Bereich durch

$$(Maximalwert-Minimalwert)/Maximalwert \times 100$$

Es hat sich überraschenderweise gezeigt, dass Lithiumdisilikat-Glaskeramiken bei ihrer Erzeugung und Vorsinterung in bestimmten Temperaturbereichen im Wesentlichen keine Veränderung der relativen Dichte und damit der linearen Schwindung und des Vergrößerungsfaktors beim Dichtsintern zeigen. Diese Bereiche sind bei der graphischen Darstellung von relativer Dichte, linearer Schwindung oder Vergrößerungsfaktor gegen die Temperatur als "Plateaus" erkennbar. Demgemäß sind für die Passgenauigkeit der späteren Dentalrestauration wichtige Eigenschaften des Rohlings in diesem Bereich im Wesentlichen unabhängig von der Temperatur. Daraus resultiert der für die Praxis wichtige Vorteil, dass der Rohling z.B. gegenüber Temperaturschwankungen oder Temperaturgradienten im Sinterofen eher unempfindlich ist, solange die Temperatur sich im Bereich des "Plateaus" befindet.

Erfindungsgemäß sind daher besonders solche vorgesinterten Rohlinge bevorzugt, die nach dem erfindungsgemäßen Verfahren erhältlich sind.

Besonders bevorzugt sind erfindungsgemäße Rohlinge, die eine relative Dichte aufweisen, die sich ergibt, wenn

(a) Pulver eines entsprechenden Ausgangsglases mit einer mittleren Teilchengröße von < 100 $\mu$m, bezogen auf die Anzahl der Teilchen, bei einem Druck von 20 bis 200 MPa, bevorzugt 40 bis 120 MPa und besonders bevorzugt 50 bis 100 MPa uniaxial oder isostatisch verpresst und

(b) der erhaltene Glaspulverpressling für 2 bis 120 min, bevorzugt 5 bis 60 min und besonders bevorzugt 10 bis 30 min bei einer Temperatur wärmebehandelt wird, die

(i) mindestens 500°C, insbesondere mindestens 540°C und bevorzugt mindestens 580°C beträgt, und

(ii) in einem Bereich liegt, der sich über mindestens 30K, insbesondere mindestens 50K und bevorzugt mindestens 70K erstreckt und in dem die relative Dichte um weniger als 2.5 %, insbesondere weniger als 2.0 % und bevorzugt weniger als 1.5 % variiert.

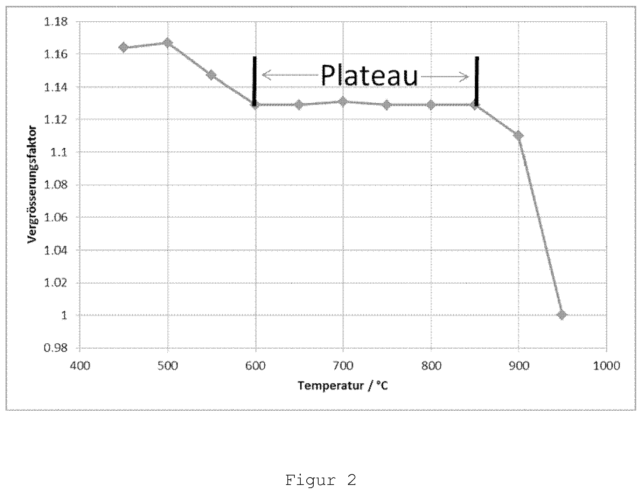

Figur 2 veranschaulicht die bei Wärmebehandlung von einem Glaspulverpressling üblicherweise durchlaufenen Phasen, indem für einen Pressling mit einer Zusammensetzung gemäß Beispiel 2 der Vergrößerungsfaktor gegen die Temperatur aufgetragen ist. In der Phase I bis etwa 500°C erfolgen das Aufheizen und die Entfernung eventuell vorhandenen Bindemittels. In der Phase II von etwa 500 bis 600°C erfolgt Sintern und Kristallisation, und in Phase III, dem Plateau, von etwa 600 bis etwa 850°C liegt ein erfindungsgemäßer vorgesinterter Rohling auf Basis von Lithiumdisilikat-Glaskeramik vor. Anschließend erfolgt in Phase IV ab etwa 850 bis etwa 950°C die Dichtsinterung des Rohlings.

Der erfindungsgemäße vorgesinterte Rohling liegt vorzugsweise in Form von Blöcken, Scheiben oder Zylindern vor. In diesen Formen ist eine Weiterverarbeitung zu den gewünschten Dentalrestaurationen besonders einfach.

Der vorgesinterte Rohling wird insbesondere zu Dentalrestaurationen weiterverarbeitet. Die Erfindung betrifft daher auch ein Verfahren zur Herstellung von Dentalrestaurationen, bei dem

(i) der erfindungsgemäße vorgesinterte Rohling auf Basis von Lithiumdisilikat-Glaskeramik durch maschinelle Bearbeitung zu einer Vorstufe der Dentalrestauration geformt wird,

(ii) die Vorstufe im Wesentlichen dichtgesintert wird, um die Dentalrestauration zu ergeben, und

(iii) gegebenenfalls die Oberfläche der Dentalrestauration endbehandelt wird.

In Stufe (i) erfolgt die maschinelle Bearbeitung üblicherweise durch materialabtragende Verfahren und insbesondere durch Fräsen und/oder Schleifen. Es ist bevorzugt, dass die maschinelle Bearbeitung mit computer-gesteuerten Fräs- und/oder Schleifvorrichtungen erfolgt. Besonders bevorzugt erfolgt die maschinelle Bearbeitung im Rahmen eines CAD/CAM-Verfahrens.

Der erfindungsgemäße Rohling kann insbesondere aufgrund seiner Offenporigkeit und geringen Festigkeit sehr einfach maschinell bearbeitet werden. Dabei ist es von besonderem Vorteil, dass der Einsatz von Flüssigkeiten während des Schleifens oder Fräsens nicht erforderlich ist. Bei konventionellen Rohlingen sind hingegen häufig sogenannte Nass-Schleifverfahren nötig.

Die maschinelle Bearbeitung wird regelmäßig so durchgeführt, dass die erhaltene Vorstufe eine vergrößerte Form der gewünschten Dentalrestauration darstellt. Dadurch wird die beim anschließenden Dichtsintern auftretende Schwindung berücksichtigt. Der erfindungsgemäße Rohling weist dabei den besonderen Vorteil auf, dass der bei ihm anzuwendende Vergrößerungsfaktor sehr genau bestimmt werden kann. Der Vergrößerungsfaktor ist der Faktor, um den die Vorstufe vergrößert aus dem vorgesinterten Rohling geschliffen oder gefräst werden muss, damit nach dem Dichtsintern die erhaltene Dentalrestauration die gewünschten Abmessungen hat.

Der Vergrößerungsfaktor $F_v$, die relative Dichte $\rho_r$ und die verbleibende lineare Schwindung S können wie folgt ineinander umgerechnet werden:

$$S = 1 - \rho_r^{1/3}$$

$$F_v = 1 / (1-S)$$

In einer bevorzugten Ausführungsform wird als vorgesinterter Rohling der nach dem oben beschriebenen erfindungsgemäßen Verfahren erzeugte Rohling eingesetzt.

In der Stufe (ii) wird die erhaltene Vorstufe im Wesentlichen dichtgesintert, um die Dentalrestauration mit der gewünschten Geometrie zu erzeugen.

Zum Dichtsintern wird die Vorstufe bevorzugt bei einer Temperatur von 700°C bis 1000°C wärmebehandelt. Die Wärmebehandlung erfolgt üblicherweise für eine Dauer von 2 bis 30 min.

Es liegt nach dem Dichtsintern eine Dentalrestauration auf Basis von Lithiumdisilikat-Glaskeramik vor, in der Lithiumdisilikat bevorzugt die Hauptkristallphase bildet. Diese Lithiumdisilikat-Glaskeramik verfügt über ausgezeichnete optische und mechanische Eigenschaften sowie eine hohe chemische Stabilität. Somit können mit dem erfindungsgemäßen Verfahren Dentalrestaurationen hergestellt werden, die hohen Anforderungen gerecht werden.

Die Dentalrestaurationen sind bevorzugt ausgewählt aus Kronen, Abutments, Abutmentkronen, Inlays, Onlays, Veneers, Schalen und Brücken sowie Überwürfen für mehrteilige Restaurationsgerüste, die z.B. aus Oxidkeramik, Metallen oder Dentallegierungen bestehen können.

Für das Dichtsintern kann es vorteilhaft sein, dass die Vorstufe der Dentalrestauration abgestützt wird, um einen Verzug zu vermeiden. Es ist bevorzugt, dass die Abstützung aus dem gleichen Material wie die Vorstufe besteht und damit den gleichen Sinterschrumpf aufweist. Die Abstützung kann z.B. in Form einer Stützkonstruktion oder Stützform vorliegen, die von ihrer Geometrie an die Vorstufe angepasst ist.

In der optionalen Stufe (iii) kann die Oberfläche der Dentalrestauration noch endbehandelt werden. Dabei ist es insbesondere möglich, noch einen Glanzbrand bei einer Temperatur von 650°C bis 900°C durchzuführen oder die Restauration zu polieren.

Aufgrund der geschilderten Eigenschaften des erfindungsgemäßen vorgesinterten Rohlings eignet er sich insbesondere zur Erzeugung von Dentalrestaurationen. Die Erfindung betrifft daher auch die Verwendung des Rohlings zur Herstellung von Dentalrestaurationen und insbesondere von Kronen, Abutments, Abutmentkronen, Inlays, Onlays, Veneers, Schalen und Brücken sowie Überwürfen.

Die angegebenen mittleren Teilchengrößen, bezogen auf die Anzahl der Teilchen, wurden durch Laserbeugung mit dem CILAS® Particle Size Analyzer 1064 der Firma Quantachrome GmbH & Co. KG nach ISO 13320 (2009) bei Raumtemperatur bestimmt.

Die Erfindung wird im Folgenden anhand von Beispielen näher erläutert.

**Beispiele**

Beispiele 1 bis 4

Es wurden insgesamt 4 Glaskeramiken mit Lithiumdisilikat als Hauptkristallphase mit der in Tabelle I angegebenen Zusammensetzung hergestellt, indem entsprechende Ausgangsgläser erschmolzen und anschließend daraus erzeugte verpresste Glaspulverrohlinge durch Wärmebehandlung vorgesintert und gesteuert kristallisiert wurden.

Dazu wurden zunächst die Ausgangsgläser im 100 bis 200 g Maßstab aus üblichen Rohstoffen bei 1400 bis 1500°C erschmolzen, wobei das Erschmelzen sehr gut ohne Bildung von Blasen oder Schlieren möglich war. Durch Eingießen der Ausgangsgläser in Wasser wurden Glasfritten hergestellt, die zur Homogenisierung anschließend ein zweites Mal bei 1450 bis 1550°C für 1 bis 3 h geschmolzen wurden.

Die erhaltenen Glasschmelzen wurden dann auf 1400°C abgekühlt und durch Eingießen in Wasser zu feinteiligen Granulaten umgewandelt. Die Granulate wurde getrocknet und zu Pulver mit einer mittleren Teilchengröße von < 100 μm, bezogen auf die Anzahl der Teilchen, gemahlen. Diese Pulver wurden mit etwas Wasser befeuchtet und bei einem Pressdruck von 20 bis 200 MPa zu Pulverpresslingen verpreßt.

Die Pulverpresslinge wurden dann für 2 bis 120 min bei einer Temperatur wärmebehandelt, die in dem Bereich liegt, der in der Tabelle I für die jeweilige Zusammensetzung als Plateau angegeben ist. Nach dieser Wärmebehandlung lagen erfindungsgemäße Rohlinge vor, die vorgesintert und auf Basis von Lithiumdisilikat-Glaskeramik waren.

Tabelle I

| Beispiel | 1 | 2 | 3 | 4 |
|---|---|---|---|---|
| Komponente | Gew.-% | Gew.-% | Gew.-% | Gew.-% |
| $SiO_2$ | 67.5 | 75.2 | 78.4 | 69.4 |
| $Li_2O$ | 14.9 | 15.6 | 16.3 | 19.7 |
| $P_2O_5$ | 4.3 | - | 3.3 | 3.4 |
| $K_2O$ | 4.2 | - | - | - |
| MgO | 0.7 | - | - | - |
| SrO | - | 4.1 | - | - |
| ZnO | 4.8 | - | - | - |
| $Al_2O_3$ | - | 3.6 | - | 3.5 |
| $La_2O_3$ | 1.0 | - | - | - |
| $Er_2O_3$ | - | 0.25 | - | - |
| $CeO_2$ | 2.0 | 1.0 | - | - |
| $SnO_2$ | - | - | 2.0 | - |
| $Nb_2O_5$ | - | - | - | - |
| $V_2O_5$ | 0.1 | - | - | - |
| $MoO_3$ | - | - | - | 4.0 |
| $Tb_4O_7$ | 0.5 | - | - | - |
| Hauptkristallphase | LS2 | LS2 | LS2 | LS2 |
| Plateau (°C) | 600 - 800 | 590 - 860 | 600 - 900 | 600 - 900 |
| LS2 Lithiumdisilikat | | | | |

Beispiel 5 - Untersuchung des Sinterverhaltens der Zusammensetzung gemäß Beispiel 1

Es wurde ein Glas mit der Zusammensetzung gemäß Beispiel 1 erschmolzen und zu einem Glaspulver mit einer mittleren Teilchengröße von weniger als 50 μm, bezogen auf die Anzahl der Teilchen, gemahlen. Dieses Glaspulver wurde zu Zylindern verpresst. Das Sinterverhalten dieser zylindrischen Rohlinge wurde untersucht, indem sie bei verschiedenen Temperaturen in einem Ofen des Typs Programat® P500 von Ivoclar Vivadent AG wärmebehandelt wurden. Dabei wurde jeweils eine Aufheizrate von 20°C/min sowie eine Haltezeit von 2 min bei der jeweiligen Temperatur benutzt. Danach wurden die Rohlinge auf Raumtemperatur abgekühlt, und es wurde dann jeweils die relative Dichte der Rohlinge in Bezug auf die Reindichte der Glaskeramik bestimmt. Aus der relativen Dichte wurden die verbleibende lineare Schwindung sowie der Vergrößerungsfaktor berechnet.

Die Ergebnisse für Temperaturen im Bereich von 25 bis 900°C sind in der folgenden Tabelle II dargestellt. Zwischen 600°C bis 800°C lag ein erfindungsgemäßer vorgesinterter Rohling aus Lithiumdisilikat-Glaskeramik mit einer relativen Dichte von 69 bis 70 % vor.

Tabelle II

| Temperatur [°C] | 25 | 450 | 500 | 550 | 600 | 650 | 700 | 750 | 800 | 850 | 900 |
|---|---|---|---|---|---|---|---|---|---|---|---|
| Durchmesser [mm] | 16.10 | 16.09 | 16.07 | 15.47 | 14.55 | 14.59 | 14.61 | 14.56 | 14.48 | 13.39 | 13.12 |
| Höhe [mm] | 15.16 | 15.14 | 15.05 | 14.91 | 13.81 | 13.80 | 13.83 | 13.88 | 13.92 | 12.65 | 12.03 |
| Volumen [cm3] | 3.09 | 3.08 | 3.05 | 2.80 | 2.30 | 2.31 | 2.32 | 2.31 | 2.29 | 1.78 | 1.63 |
| Masse [g] | 4.00 | 3.98 | 3.99 | 4.05 | 3.98 | 3.98 | 3.97 | 3.99 | 4.01 | 4.03 | 3.98 |
| Dichte [g/cm3] | 1.30 | 1.29 | 1.31 | 1.45 | 1.74 | 1.73 | 1.71 | 1.73 | 1.75 | 2.26 | 2.45 |

7

(fortgesetzt)

| Temperatur [°C] | 25 | 450 | 500 | 550 | 600 | 650 | 700 | 750 | 800 | 850 | 900 |
|---|---|---|---|---|---|---|---|---|---|---|---|
| relative Dichte [%] | 52 | 52 | 52 | 58 | 69 | 69 | 69 | 69 | 70 | 91 | 98 |
| lineare Schwindung [%] | 19.6 | 19.5 | 19.2 | 17.3 | 11.4 | 11.5 | 11.6 | 11.6 | 11.5 | 3.5 | 0.0 |
| Vergrösserungsfaktor | 1.24 | 1.24 | 1.24 | 1.21 | 1.13 | 1.13 | 1.13 | 1.13 | 1.13 | 1.04 | 1.00 |

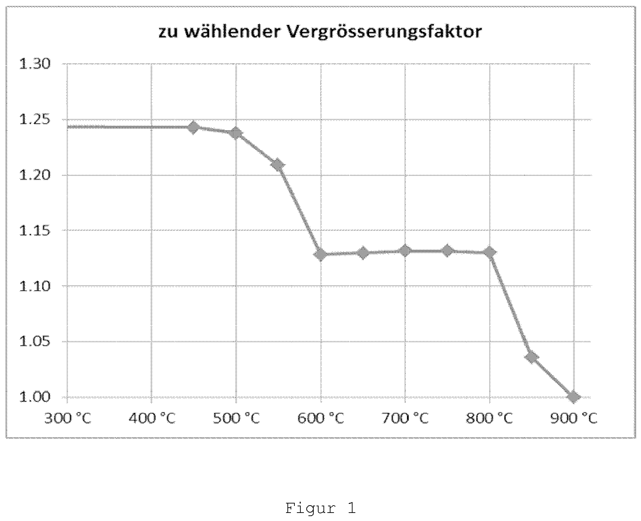

Die graphische Darstellung des berechneten Vergrößerungsfaktors gegen die angewendete Temperatur ist in Figur 1 gezeigt. Es ist daraus ersichtlich, dass der Vergrößerungsfaktor überraschenderweise im Bereich von 600 bis 800°C im Wesentlichen konstant bleibt und die Kurve ein Plateau bildet. Somit ist bei der Anwendung einer Wärmebehandlung in diesem Bereich ein erfindungsgemäßer Rohling erzeugbar, für den eine sehr genaue Angabe des zu wählenden Vergrößerungsfaktors möglich ist.

In gleicher Weise wurde bei den anderen in Tabelle I genannten Zusammensetzungen verfahren, um jeweils diesen Bereich ("Plateau") zu bestimmen.

Beispiel 6 - Untersuchung des Sinterverhaltens der Zusammensetzung gemäß Beispiel 2

An der Zusammensetzung gemäß Beispiel 2 wurde analog Beispiel 5 das Sinterverhalten untersucht. Die erhaltenen Werte für die relative Dichte, die verbleibende lineare Schwindung und den Vergrösserungsfaktor sind in Tabelle III aufgeführt.

Tabelle III

| Temperatur [°C] | 30 | 450 | 500 | 550 | 600 | 650 | 700 | 750 | 800 | 850 | 900 | 950 |
|---|---|---|---|---|---|---|---|---|---|---|---|---|
| Lineare Schwindung [%] | 14.05 | 14.10 | 14.30 | 12.80 | 11.45 | 11.45 | 11.55 | 11.45 | 11.40 | 11.45 | 9.95 | 0.00 |
| Vergrösserungsfaktor | 1.163 | 1.164 | 1.167 | 1.147 | 1.129 | 1.129 | 1.131 | 1.129 | 1.129 | 1.129 | 1.110 | 1.000 |
| Relative Dichte [%] | 63 | 63 | 63 | 66 | 69 | 69 | 69 | 69 | 70 | 70 | 73 | 100 |

Der Vergrößerungsfaktor wurde gegen die Temperatur aufgetragen und die dabei erhaltene Kurve ist in Figur 2 gezeigt. Aus ihr ist ersichtlich, dass sich das Plateau für die untersuchte Lithiumdisilikat-Glaskeramik in einem Bereich von etwa 600 bis etwa 850°C befindet. In diesem Bereich liegt ein erfindungsgemäßer vorgesinterter Rohling aus Lithiumdisilikat-Glaskeramik mit einer relativen Dichte von 69 bis 70 % vor.

**Patentansprüche**

1. Vorgesinterter Rohling für dentale Zwecke auf Basis von Lithiumdisilikat-Glaskeramik, wobei der Rohling eine relative Dichte von 60 bis 90%, insbesondere 62 bis 88% und bevorzugt 65 bis 87 %, bezogen auf die Reindichte der Glaskeramik aufweist.

2. Rohling nach Anspruch 1, der im Wesentlichen aus der Lithiumdisilikat-Glaskeramik besteht.

3. Rohling nach einem der Ansprüche 1 bis 2, wobei die Glaskeramik Lithiumdisilikat als Hauptkristallphase aufweist und insbesondere mehr als 10 Vol.-%, bevorzugt mehr als 20 Vol.-% und besonders bevorzugt mehr als 30 Vol.-% an Lithiumdisilikat-Kristallen enthält.

4. Rohling nach einem der Ansprüche 1 bis 3, wobei die Lithiumdisilikat-Glaskeramik mindestens eine der folgenden Komponenten enthält:

   | Komponente | Gew.-% |
   |---|---|
   | $SiO_2$ | 50,0 bis 80,0 |
   | $Li_2O$ | 6,0 bis 20,0 |
   | $Me(I)_2O$ | 0 bis 10,0, insbesondere 0,1 bis 10,0 |
   | $Me(II)O$ | 0 bis 12,0, insbesondere 0,1 bis 12,0 |
   | $Me(III)_2O_3$ | 0 bis 8,0, insbesondere 0,1 bis 8,0 |
   | $Me(IV)O_2$ | 0 bis 8,0, insbesondere 0,1 bis 8,0 |
   | $Me(V)_2O_5$ | 0 bis 8,0, insbesondere 0,1 bis 8,0 |
   | $Me(VI)O_3$ | 0 bis 8,0, insbesondere 0,1 bis 8,0 |
   | Keimbildner | 0 bis 8,0, insbesondere 0,1 bis 8,0, |

   wobei

   $Me(I)_2O$ ausgewählt ist aus $Na_2O$, $K_2O$, $Rb_2O$, $Cs_2O$ oder Mischungen davon,
   $Me(II)O$ ausgewählt ist aus $CaO$, $BaO$, $MgO$, $SrO$, $ZnO$ und Mischungen davon,
   $Me(III)_2O_3$ ausgewählt ist aus $Al_2O_3$, $La_2O_3$, $Bi_2O_3$, $Y_2O_3$, $Yb_2O_3$ und Mischungen davon,
   $Me(IV)O_2$ ausgewählt ist aus $ZrO_2$, $TiO_2$, $SnO_2$, $GeO_2$ und Mischungen davon,
   $Me(V)_2O_5$ ausgewählt ist aus $Ta_2O_5$, $Nb_2O_5$ und Mischungen davon,
   $Me(VI)O_3$ ausgewählt ist aus $WO_3$, $MoO_3$ und Mischungen davon, und
   Keimbildner ausgewählt ist aus $P_2O_5$, Metallen und Mischungen davon.

5. Rohling nach einem der Ansprüche 1 bis 4, der mindestens zwei Bereiche, insbesondere Schichten, aufweist, die sich durch ihre Färbung oder Transluzenz unterscheiden.

6. Rohling nach einem der Ansprüche 1 bis 5, der einen Halter für eine Bearbeitungsvorrichtung aufweist.

7. Rohling nach einem der Ansprüche 1 bis 6, der eine Schnittstelle, insbesondere in Form einer Ausnehmung, zur Verbindung mit einem Dentalimplantat aufweist.

8. Rohling nach einem der Ansprüche 1 bis 7, der durch das Verfahren nach einem der Ansprüche 10 oder 11 erhältlich ist.

9. Rohling nach einem der Ansprüche 1 bis 8, der eine relative Dichte aufweist, die sich ergibt, wenn

(a) Pulver eines entsprechenden Ausgangsglases mit einer mittleren Teilchengröße von < 100 μm, bezogen auf die Anzahl der Teilchen, bei einem Druck von 20 MPa bis 200 MPa, bevorzugt 40 bis 120 MPa und besonders bevorzugt 50 bis 100 MPa uniaxial oder isostatisch verpresst und

(b) der erhaltene Glaspulverpressling für 2 bis 120 min, bevorzugt 5 bis 60 min und besonders bevorzugt 10 bis 30 min bei einer Temperatur wärmebehandelt wird, die

(i) mindestens 500°C, insbesondere mindestens 540°C und bevorzugt mindestens 580°C beträgt, und

(ii) in einem Bereich liegt, der sich über mindestens 30K, insbesondere mindestens 50K und bevorzugt mindestens 70K erstreckt und in dem die relative Dichte um weniger als 2.5 %, insbesondere weniger als 2.0 % und bevorzugt weniger als 1.5 % variiert.

10. Verfahren zur Herstellung des Rohlings nach einem der Ansprüche 1 bis 7 oder 9, bei dem

(a) Lithiumsilikat-Glas in Pulver- oder Granulatform zu einem Glasrohling verpresst wird,

(b) der Glasrohling wärmebehandelt wird, um einen vorgesinterten Rohling auf Basis von Lithiumdisilikat-Glaskeramik herzustellen, wobei die Temperatur der Wärmebehandlung

(i) mindestens 500°C, insbesondere mindestens 540°C und bevorzugt mindestens 580°C beträgt, und

(ii) in einem Bereich liegt, der sich über mindestens 30K, insbesondere mindestens 50K und bevorzugt mindestens 70K erstreckt und in dem die relative Dichte um weniger als 2.5 %, insbesondere weniger als 2.0 % und bevorzugt weniger als 1.5 % variiert.

11. Verfahren nach Anspruch 10, bei dem in Schritt (a) mindestens zwei Lithiumsilikat-Gläser eingesetzt werden, die sich durch ihre Färbung oder Transluzenz unterscheiden.

12. Verfahren zur Herstellung von Dentalrestaurationen, bei dem

(i) der vorgesinterte Rohling auf Basis von Lithiumdisilikat-Glaskeramik nach einem der Ansprüche 1 bis 9 durch maschinelle Bearbeitung zu einer Vorstufe der Dentalrestauration geformt wird,

(ii) die Vorstufe im Wesentlichen dichtgesintert wird, um die Dentalrestauration zu ergeben, und

(iii) gegebenenfalls die Oberfläche der Dentalrestauration endbehandelt wird.

13. Verfahren nach Anspruch 12, bei dem die maschinelle Bearbeitung mit computergesteuerten Fräs- und/oder Schleifvorrichtungen erfolgt.

14. Verfahren nach Anspruch 12 oder 13, bei dem das Verfahren gemäß Anspruch 10 oder 11 durchgeführt wird, um den vorgesinterten Rohling auf Basis von Lithiumdisilikat-Glaskeramik zu erhalten.

15. Verfahren nach einem der Ansprüche 12 bis 14, bei dem die Dentalrestaurationen ausgewählt sind aus Kronen, Abutments, Abutmentkronen, Inlays, Onlays, Veneers, Schalen, Brücken und Überwürfen.

16. Verwendung des Rohlings nach einem der Ansprüche 1 bis 9 zur Herstellung von Dentalrestaurationen und insbesondere von Kronen, Abutments, Abutmentkronen, Inlays, Onlays, Veneers, Schalen, Brücken und Überwürfen.

**Claims**

1. Pre-sintered blank for dental purposes based on lithium disilicate glass ceramic, wherein the blank has a relative density of from 60 to 90%, in particular 62 to 88% and preferably 65 to 87%, relative to the true density of the glass ceramic.

2. Blank according to claim 1, which consists essentially of lithium disilicate glass ceramic.

3. Blank according to any one of claims 1 to 2, wherein the glass ceramic comprises lithium disilicate as main crystal phase and in particular comprises more than 10 vol.-%, preferably more than 20 vol.-% and particularly preferably more than 30 vol.-% lithium disilicate crystals.

4. Blank according to any one of claims 1 to 3, wherein the lithium disilicate glass ceramic comprises at least one of the following components:

| Component | wt.-% |
|---|---|
| $SiO_2$ | 50.0 to 80.0 |
| $Li_2O$ | 6.0 to 20.0 |
| $Me(I)_2O$ | 0 to 10.0, in particular 0.1 to 10.0 |
| $Me(II)O$ | 0 to 12.0, in particular 0.1 to 12.0 |
| $Me(III)_2O_3$ | 0 to 8.0, in particular 0.1 to 8.0 |
| $Me(IV)O_2$ | 0 to 8.0, in particular 0.1 to 8.0 |
| $Me(V)_2O_5$ | 0 to 8.0, in particular 0.1 to 8.0 |
| $Me(VI)O_3$ | 0 to 8.0, in particular 0.1 to 8.0 |
| nucleating agent | 0 to 8.0, in particular 0.1 to 8.0, |

wherein

$Me(I)_2O$ is selected from $Na_2O$, $K_2O$, $Rb_2O$, $Cs_2O$ or mixtures thereof,
$Me(II)O$ is selected from $CaO$, $BaO$, $MgO$, $SrO$, $ZnO$ and mixtures thereof,
$Me(III)_2O_3$ is selected from $Al_2O_3$, $La_2O_3$, $Bi_2O_3$, $Y_2O_3$, $Yb_2O_3$ and mixtures thereof,
$Me(IV)O_2$ is selected from $ZrO_2$, $TiO_2$, $SnO_2$, $GeO_2$ and mixtures thereof,
$Me(V)_2O_5$ is selected from $Ta_2O_5$, $Nb_2O_5$ and mixtures thereof,
$Me(VI)O_3$ is selected from $WO_3$, $MoO_3$ and mixtures thereof, and
nucleating agent is selected from $P_2O_5$, metals and mixtures thereof.

5. Blank according to any one of claims 1 to 4, which has at least two areas, in particular layers, which differ by their coloration or translucence.

6. Blank according to any one of claims 1 to 5, which has a holder for a processing device.

7. Blank according to any one of claims 1 to 6, which has an interface, in particular in the form of a recess, for connection to a dental implant.

8. Blank according to any one of claims 1 to 7, which is obtainable by the process according to any one of claims 10 or 11.

9. Blank according to any one of claims 1 to 8, which has a relative density which results when

(a) powder of a corresponding starting glass with an average particle size of < 100 μm, relative to the number of particles, is uniaxially or isostatically pressed at a pressure of from 20 MPa to 200 MPa, preferably 40 to 120 MPa and particularly preferably 50 to 100 MPa, and
(b) the obtained glass powder green compact is heat-treated for 2 to 120 min, preferably 5 to 60 min and particularly preferably 10 to 30 min at a temperature which

(i) is at least 500°C, in particular at least 540°C and preferably at least 580°C, and
(ii) lies in a range which extends over at least 30K, in particular at least 50K and preferably at least 70K and in which the relative density varies by less than 2.5%, in particular less than 2.0% and preferably less than 1.5%.

10. Process for the preparation of the blank according to any one of claims 1 to 7 or 9, in which

(a) lithium silicate glass in powder or granulate form is pressed to form a glass blank,
(b) the glass blank is heat-treated in order to prepare a pre-sintered blank based on lithium disilicate glass ceramic, wherein the temperature of the heat treatment

(i) is at least 500°C, in particular at least 540°C and preferably at least 580°C, and
(ii) lies in a range which extends over at least 30K, in particular at least 50K and preferably at least 70K and in which the relative density varies by less than 2.5%, in particular less than 2.0% and preferably less than 1.5%.

**11.** Process according to claim 10, in which in step (a) at least two lithium silicate glasses are used which differ in terms of their coloration or translucence.

**12.** Process for the preparation of dental restorations, in which

(i) the pre-sintered blank based on lithium disilicate glass ceramic according to any one of claims 1 to 9 is shaped by machining to form a precursor of the dental restoration,
(ii) the precursor is substantially dense-sintered in order to produce the dental restoration, and
(iii) optionally the surface of the dental restoration is provided with a finish.

**13.** Process according to claim 12, in which the machining is carried out with computer-controlled milling and/or grinding devices.

**14.** Process according to claim 12 or 13, in which the process according to claim 10 or 11 is carried out in order to obtain the pre-sintered blank based on lithium disilicate glass ceramic.

**15.** Process according to any one of claims 12 to 14, in which the dental restorations are selected from crowns, abutments, abutment crowns, inlays, onlays, veneers, shells, bridges and overstructures.

**16.** Use of the blank according to any one of claims 1 to 9 to prepare dental restorations and in particular crowns, abutments, abutment crowns, inlays, onlays, veneers, shells, bridges and overstructures.


**Revendications**

**1.** Corps d'ébauche pré-fritté pour dentisterie, à base de vitrocéramique de disilicate de lithium, lequel corps d'ébauche présente une densité relative de 60 à 90 %, en particulier de 62 à 88 % et de préférence de 65 à 87 %, par rapport à la densité absolue de la vitrocéramique.

**2.** Corps d'ébauche conforme à la revendication 1, essentiellement constitué de la vitrocéramique de disilicate de lithium.

**3.** Corps d'ébauche conforme à l'une des revendications 1 et 2, dans lequel la vitrocéramique comporte du disilicate de lithium en tant que principale phase cristalline et contient en particulier plus de 10 % en volume, de préférence plus de 20 % en volume, et mieux encore plus de 30 % en volume de cristaux de disilicate de lithium.

**4.** Corps d'ébauche conforme à l'une des revendications 1 à 3, dans lequel la vitrocéramique de disilicate de lithium contient au moins l'un des composants suivants :

| Composant | Pourcentage pondéral | |
|---|---|---|
| $SiO_2$ | 50,0 à 80,0 % | |
| $Li_2O$ | 6,0 à 20,0 % | |
| $Me(I)_2O$ | 0 à 10,0 %, | en particulier 0,1 à 10,0 % |
| $Me(II)O$ | 0 à 12,0 %, | en particulier 0,1 à 12,0 % |
| $Me(III)_2O_3$ | 0 à 8,0 %, | en particulier 0,1 à 8,0 % |
| $Me(IV)O_2$ | 0 à 8,0 %, | en particulier 0,1 à 8,0 % |
| $Me(V)_2O_5$ | 0 à 8,0 %, | en particulier 0,1 à 8,0 % |
| $Me(VI)O_3$ | 0 à 8,0 %, | en particulier 0,1 à 8,0 % |
| Germinateur | 0 à 8,0 %, | en particulier 0,1 à 8,0 % |

étant entendu que

$Me(I)_2O$ est choisi parmi $Na_2O$, $K_2O$, $Rb_2O$, $Cs_2O$ et leurs mélanges,
$Me(II)O$ est choisi parmi $CaO$, $BaO$, $MgO$, $SrO$, $ZnO$ et leurs mélanges,
$Me(III)_2O_3$ est choisi parmi $Al_2O_3$, $La_2O_3$, $Bi_2O_3$, $Y_2O_3$, $Yb_2O_3$ et leurs mélanges,
$Me(IV)O_2$ est choisi parmi $ZrO_2$, $TiO_2$, $SnO_2$, $GeO_2$ et leurs mélanges,

Me(V)$_2$O$_5$ est choisi parmi Ta$_2$O$_5$, Nb$_2$O$_5$ et leurs mélanges,
Me(VI)O$_3$ est choisi parmi WO$_3$, MoO$_3$ et leurs mélanges,
et le germinateur est choisi parmi P$_2$O$_5$, les métaux, et leurs mélanges.

5. Corps d'ébauche conforme à l'une des revendications 1 à 4, qui comporte au moins deux domaines, en particulier des couches, qui se différencient par leur coloration ou leur aspect translucide.

6. Corps d'ébauche conforme à l'une des revendications 1 à 5, qui comporte un appui pour dispositif de façonnage.

7. Corps d'ébauche conforme à l'une des revendications 1 à 6, qui présente un endroit découpé, ayant en particulier la forme d'un évidement, pour liaison avec un implant dentaire.

8. Corps d'ébauche conforme à l'une des revendications 1 à 7, qui est accessible par un procédé conforme à l'une des revendications 10 et 11.

9. Corps d'ébauche conforme à l'une des revendications 1 à 8, qui présente la densité relative obtenue quand on opère comme suit :

a) comprimer de manière unidirectionnelle ou isostatique, sous une pression de 20 à 200 MPa, de préférence de 40 à 120 MPa et surtout de 50 à 100 MPa, une poudre d'un verre de départ correspondant dont les particules ont une taille, en moyenne rapportée au nombre de particules, inférieure à 100 $\mu$m,
b) et soumettre à un traitement thermique le corps de poudre de verre comprimée ainsi obtenu, durant 2 à 120 minutes, de préférence 5 à 60 minutes et surtout 10 à 30 minutes, à une température i)

qui vaut au moins 500 °C, en particulier au moins 540 °C et de préférence au moins 580 °C,
ii) et qui se situe dans un intervalle qui s'étend sur au moins 30 K, en particulier au moins 50 K et de préférence au moins 70 K, mais dans lequel la densité relative varie de moins de 2,5 %, en particulier de moins de 2,0 % et de préférence de moins de 1,5 %.

10. Procédé de fabrication d'un corps d'ébauche conforme à l'une des revendications 1 à 7 et 9, dans lequel

a) on comprime un verre de silicate de lithium se présentant sous forme de poudre ou de granulat, pour en faire un corps d'ébauche en verre,
b) et l'on soumet ce corps d'ébauche en verre à un traitement thermique, pour en faire un corps d'ébauche préfritté à base de vitrocéramique de disilicate de lithium, étant entendu que la température de ce traitement thermique

i) vaut au moins 500 °C, en particulier au moins 540 °C et de préférence au moins 580 °C,
ii) et se situe dans un intervalle qui s'étend sur au moins 30 K, en particulier au moins 50 K et de préférence au moins 70 K, mais dans lequel la densité relative varie de moins de 2,5 %, en particulier de moins de 2,0 % et de préférence de moins de 1,5 %.

11. Procédé conforme à la revendication 10, dans lequel on utilise dans l'étape (a) au moins deux verres de silicate de lithium qui se différencient par leur coloration ou leur aspect translucide.

12. Procédé de fabrication de pièces de restauration dentaire, dans lequel

i) on façonne, en opérant sur machine, un corps d'ébauche pré-fritté à base de vitrocéramique de disilicate de lithium, conforme à l'une des revendications 1 à 9, pour en faire un précurseur de pièce de restauration dentaire,
ii) on soumet ce précurseur, pour l'essentiel, à un frittage poussé, pour obtenir une pièce de restauration dentaire,
iii) et en option, on réalise un traitement de finissage de la surface de cette pièce de restauration dentaire.

13. Procédé conforme à la revendication 12, dans lequel on effectue le façonnage sur machine au moyen d'appareils de fraisage et/ou de meulage commandés par ordinateur.

14. Procédé conforme à la revendication 12 ou 13, dans lequel on met en oeuvre un procédé conforme à la revendication 10 ou 11 pour obtenir le corps d'ébauche pré-fritté à base de vitrocéramique de disilicate de lithium.

**15.** Procédé conforme à l'une des revendications 12 à 14, dans lequel les pièces de restauration dentaire sont choisies parmi les couronnes, piliers, piliers-couronnes, inserts « inlays » ou « onlays », facettes « veneer », coquilles, bridges et couvertures.

**16.** Utilisation d'un corps d'ébauche conforme à l'une des revendications 1 à 9, pour la fabrication de pièces de restauration dentaire, en particulier de couronnes, piliers, piliers-couronnes, inserts « inlays » ou « onlays », facettes « veneer », coquilles, bridges et couvertures.

Figur 1

Figur 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2010010087 A
- US 5106303 A
- US 5775912 A
- US 6354836 B